# EUROPEAN PATENT APPLICATION

(11) **EP 3 869 478 A1**
(43) Date of publication of application: **25.08.2021**
(21) Application number: 19873071.5
(22) Date of filing: 11.10.2019
(51) Int. Cl.: G08C 19/00, A61B 5/0408, A61B 5/0478, G01D 9/00

(54) **DATA ACQUISITION METHOD AND SIGNAL MEASUREMENT SYSTEM**

(30) Priority: 18.10.2018 JP 2018196787; 27.09.2019 JP 2019177231
(71) Applicant: Nitto Denko Corporation, Ibaraki-shi, Osaka 567-8680 (JP)
(72) Inventor: YOSHIOKA, Ryoma, Ibaraki-shi, Osaka 567-8680 (JP); MORI, Shigeyasu, Ibaraki-shi, Osaka 567-8680 (JP)
(74) Representative: Hoffmann Eitle
(86) International application number: PCT/JP2019/040243
(87) International publication number: WO 2020/080296

(57) **Abstract**

A data acquisition method and a signal measurement system that allow measured data to be easily and reliably obtained from memory are provided. The signal measurement system includes a reception device on which a plurality of devices storing measurement data are placed, a probe mechanism configured to be able to come into contact with the plurality of devices placed on the reception device, a control circuit configured to read, in parallel, the measurement data from the plurality of devices with the probe mechanism, and an information processing apparatus configured to process, in parallel, the measurement data having been read, wherein at a point in time when a subsequent device set is placed on the reception device, the control circuit drives the probe mechanism, and at a point in time when data processing with the information processing apparatus is completed, the control circuit starts reading, in parallel, data from the subsequent device set.

## Description

### TECHNICAL FIELD

The present invention relates to a data acquisition method and a signal measurement system.

### BACKGROUND ART

Conventionally, an adhering-type biosensor pasted to the surface of a living body to measure the living body has been known. As such a biosensor, for example, a biocompatible polymer substrate including a data acquisition module, a polymer layer having viscosity, an electrode arranged on the polymer layer, and a wiring connecting the data acquisition module and the electrode has been suggested (for example, see PTL 1) .

In such a biocompatible polymer substrate, the polymer layer is pasted to the surface of the living body, and the electrode detects a bio-signal, for example, a myocardial voltage signal, and the data acquisition module receives and records the myocardial voltage signal.

### SUMMARY OF THE INVENTION

### [Technical Problem]

Patent Literature 1 indicates that a protective material such as a polymer layer is arranged on an upper surface of a biocompatible polymer substrate so as to cover the data acquisition module. In this case, in order to prevent the mounted components such as the data acquisition module from coming off and to provide waterproofness, the mounted components may be completely sealed and bonded with the protective material.

However, in that case, the protective material is bonded to the polymer substrate or the modules, and therefore, when the data (memory) is retrieved after the measurement, it is necessary to peel off the protective material to expose the module, and it is not easy to retrieve the data (memory) in the module.

A method of transmitting the bio-data in the module to an external display to retrieve the bio-data by a wireless method is also considered. However, the amount of bio-data is enormous, and therefore, there is a problem in that it takes a long communication time. In addition, in medical applications, higher communication accuracy is required from the viewpoint of safety. Therefore, the acquisition by the wireless method is less reliable.

The present invention provides a data acquisition method and a signal measurement system that allow measured data to be easily and reliably obtained from memory.

### [Solution to Problem]

The present invention [1] includes a data acquisition method for obtaining data from a sensor including a sensor unit configured to sense a physical or electric signal, memory configured to store, as the data, the physical or electric signal from the sensor unit, a terminal configured to output the data in the memory, and a cover covering the memory and the terminal,
the data acquisition method including:
preparing an external apparatus including a probe for retrieving the data from the memory; and
retrieving the data stored in the memory by bringing the probe of the external apparatus in contact with the terminal.

According to this data acquisition method, the data in the memory is obtained by bringing the probe of the external apparatus in contact with the terminal, so that the measured data can be obtained easily. In addition, the wired method in which the probe is in contact with the terminal is adopted, so that the communication speed and communication accuracy are better than those of the wireless method. Therefore, the data can be obtained reliably.

According to another aspect of the present invention, a signal measurement system includes:
a reception device on which a plurality of devices storing measurement data are placed;
a probe mechanism configured to be able to come into contact with the plurality of devices placed on the reception device;
a control circuit configured to read, in parallel, the measurement data from the plurality of devices with the probe mechanism; and
an information processing apparatus configured to process, in parallel, the measurement data having been read,
wherein at a point in time when a subsequent device set is placed on the reception device, the control circuit drives the probe mechanism, and
at a point in time when data processing with the information processing apparatus is completed, the control circuit starts reading, in parallel, data from the subsequent device set.

According to this signal measurement system, the waiting time is minimized, and a highspeed data reading and data processing can be achieved.

In a preferred configuration example, the reception device generates and outputs information about a placement state of the plurality of devices, and
the control circuit drives the probe mechanism based on the information.

Accordingly, immediately after the subsequent device set is placed, the probe of the probe mechanism can be brought into contact with the corresponding device.

For example, one of the plurality of devices is a biosensor configured to sense a signal from a living body by being brought into contact with the living body, and
the biosensor further includes an alignment unit serving as a reference of alignment of the probe mechanism with respect to the biosensor.

According to this data acquisition method, the living body can be measured, and the data derived from the living body can be obtained easily.

### [Advantageous Effects of Invention]

According to the data acquisition method and the signal measurement system of the present invention, measured data can be easily and reliably obtained from memory.

### BRIEF DESCRIPTION OF THE DRAWINGS

FIG. 1A is a plan view of an adhering-type biosensor used for a signal measurement system according to a first embodiment;
FIG. 1B is a cross-sectional view taken along line A-A of FIG. 1A;
FIG. 2A illustrates an aspect of data acquisition of a bio-signal measurement system according to the first embodiment;
FIG. 2B is a drawing illustrating an aspect in which probes are brought into contact with terminals;
FIG. 3 illustrates a modified embodiment of data acquisition (an aspect having an alignment unit);
FIG. 4 illustrates another modified embodiment of data acquisition (an aspect in which multiple probes are brought into contact with a single terminal);
FIG. 5 illustrates still another modified embodiment of data acquisition (an aspect in which probes penetrate through terminals);
FIG. 6A illustrates a modified embodiment of an external reception apparatus of a bio-signal measurement system;
FIG. 6B illustrates another modified embodiment of an external reception apparatus of a bio-signal measurement system;
FIG. 7A illustrates a modified embodiment of probes of the external reception apparatus;
FIG. 7B illustrates another modified embodiment of probes of the external reception apparatus;
FIG. 8 is a schematic diagram of a biosensor according to a second embodiment;
FIG. 9 is a flowchart of a data acquisition method according to the second embodiment;
FIG. 10A illustrates parallel processing during data acquisition according to the second embodiment;
FIG. 10B illustrates generally-available sequential processing as a comparative example;
FIG. 11 is a schematic diagram of the bio-signal measurement system according to the second embodiment;
FIG. 12 is a schematic diagram of probes used in the second embodiment; and
FIG. 13 is a schematic diagram of a probe mechanism for performing parallel reading.

### MODES FOR CARRYING OUT THE INVENTION

### <First embodiment>

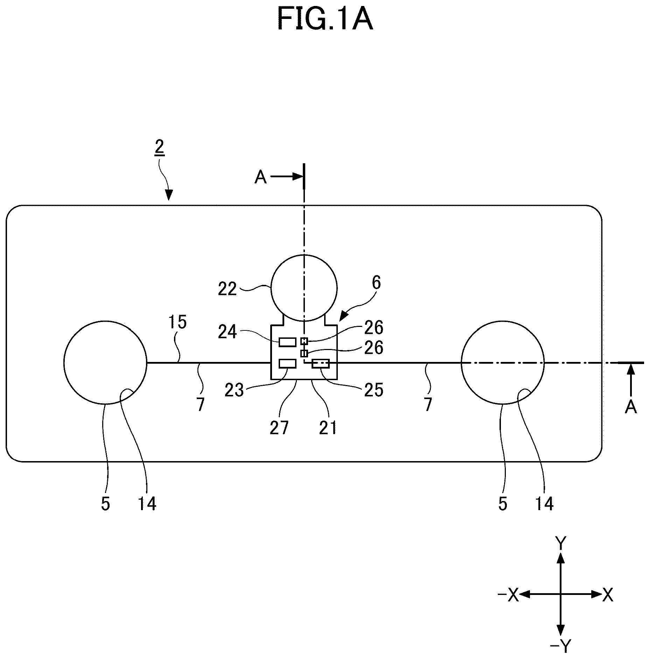

As an embodiment of a signal measurement system and a method for using the same according to the present invention, a bio-signal measurement system and a method for using the same are explained with reference to FIG. 1A to FIG. 2B.

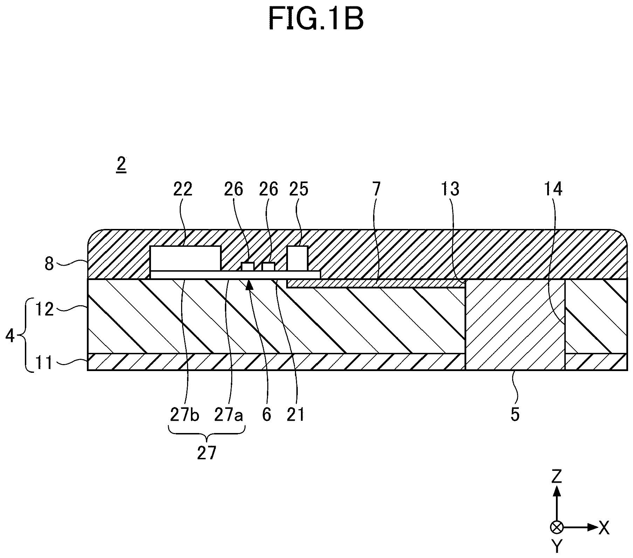

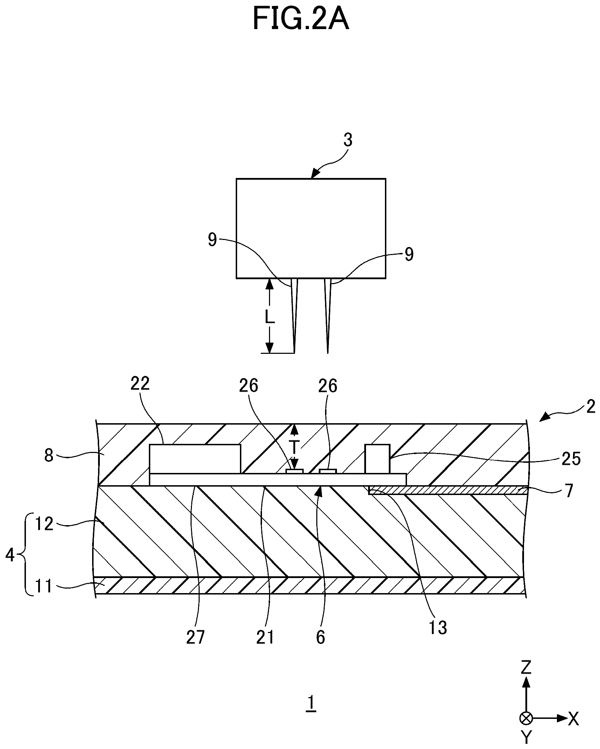

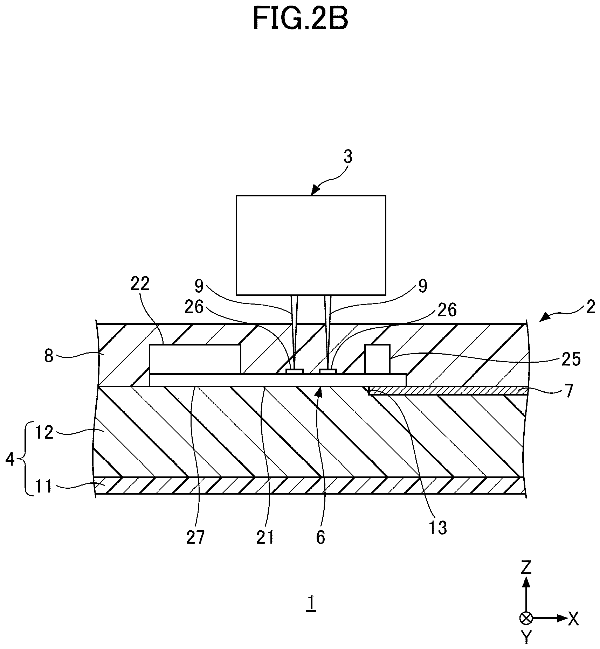

FIG. 1A is a plan view of an adhering-type biosensor 2 used in a bio-signal measurement system according to the first embodiment, and illustrates a configuration in the X-Y plane. FIG. 1B is a cross-sectional view taken along line A-A of FIG. 1A. The thickness direction of the biosensor 2 is defined as a Z direction orthogonal to the X-Y plane. Both of FIG. 2A and FIG. 2B are cross-sectional views taken along a film thickness direction, i.e., a Z direction. For the sake of convenience of illustration, a coating layer is omitted from FIG. 1A.

### 1. Bio-signal measurement system

As illustrated in FIG. 2 and FIG. 2B, a bio-signal measurement system 1 includes an adhering-type biosensor 2 (an example of a sensor) and an external reception apparatus 3. Hereinafter, they are explained in detail.

### 2. Biosensor

As illustrated in FIG. 1A and FIG. 1B, the adhering-type biosensor 2 is configured as a patch pasted to a living body and as a measurement device for measuring a signal from the living body. The adhering-type biosensor 2 is a sheet extending in the X-Y plane, and has, for example, a substantially rectangular shape that is longer in the X direction in the plan view.

The adhering-type biosensor 2 includes a sensor substrate 4, sensor units 5, a battery-attached control unit 6, a sensor wiring unit 7, and a coating layer 8.

(Sensor substrate) The sensor substrate 4 is a flexible member for supporting the sensor units 5, the battery-attached control unit 6, and the sensor wiring unit 7. The sensor substrate 4 forms an external shape of the adhering-type biosensor 2. The sensor substrate 4 has elasticity and pressure-sensitive adhesiveness.

The sensor substrate 4 includes a pressure-sensitive adhesive layer 11 and a substrate layer 12 arranged on an upper surface (one of the surfaces in the thickness direction) of the pressure-sensitive adhesive layer 11.

The pressure-sensitive adhesive layer 11 constitutes the lower surface (pressure-sensitive adhesive surface) of the sensor substrate 4. The pressure-sensitive adhesive layer 11 is a layer for giving pressure-sensitive adhesiveness to the lower surface of the adhering-type biosensor 2 in order to paste the sensor substrate 4 to the living body.

Examples of materials of the pressure-sensitive adhesive layer 11 include a material having biocompatibility. Examples of such materials include acryl-based pressure-sensitive adhesive agents and silicone-based pressure-sensitive adhesive agents, and the pressure-sensitive adhesive layer 11 is preferably made of an acryl-based pressure-sensitive adhesive agent. Examples of the acryl-based pressure-sensitive adhesive agents include an acrylic polymer described in Japanese Laid-Open Patent Publication No. 2003-342541.

The thickness of the pressure-sensitive adhesive layer 11 is, for example, 10 µm or more, preferably 20 µm or more, and is, for example, 95 µm or less, preferably 70 µm or less.

The substrate layer 12 constitutes the upper surface of the sensor substrate 4. The substrate layer 12 is a layer that constitutes, together with the pressure-sensitive adhesive layer 11, the outer shape of the sensor substrate 4 and that supports the pressure-sensitive adhesive layer 11. The shape of the substrate layer 12 in the plan view is substantially the same as the shape of the pressure-sensitive adhesive layer 11 in the plan view. The substrate layer 12 is arranged on the entirety of the upper surface of the pressure-sensitive adhesive layer 11.

Examples of materials of the substrate layer 12 include an insulator having elasticity. Examples of such materials include polyurethane-based resins, silicone-based resins, acryl-based resins, polystyrene-based resins, vinyl chloride-based resins, polyester-based resins, and the like, and the substrate layer 12 is preferably made of a polyurethane-based resin.

The thickness of the substrate layer 12 is, for example, 1 µm or more, preferably 5 µm or more, and is, for example, 95 µm or less, preferably 50 µm or less.

The substrate layer 12 includes substrate grooves 13 corresponding to the sensor wiring unit 7. The substrate grooves 13 have the same shape and the same dimensions as the sensor wiring unit 7.

The sensor substrate 4 includes multiple (two) through holes 14 corresponding to the sensor units 5. The through holes 14 are formed at respective end portions of the sensor substrate 4 in the second direction, with one being at each end, and have approximately circular shape in the plan view.

The dimensions of the sensor substrate 4 in the plan view are set as appropriate in accordance with a portion of the living body to which the adhering-type biosensor 2 is pasted. The length of the sensor substrate 4 in the Y direction is, for example, 5 mm or more, preferably 10 mm or more, and is, for example, 300 mm or less, preferably 100 mm or less. The length of the sensor substrate 4 in the X direction is, for example, 30 mm or more, preferably 50 mm or more, and is, for example, 1000 mm or less, preferably 200 mm or less.

### (Sensor unit)

The sensor units 5 are electrodes (bioelectrodes) for sensing a signal of the living body (an electrical signal or a physical signal) such as, for example, an electric signal, temperature, vibration, sweat, metabolites, and the like, by coming into contact with the skin of the living body when the pressure-sensitive adhesive layer 11 is pasted to the skin of the living body.

Multiple (two) sensor units 5 are arranged. The multiple sensor units 5 are arranged in the inner portions of the respective through holes 14 of the sensor substrate 4. Specifically, the sensor units 5 are embedded in the substrate layer 12 so as to be exposed from the lower surface of the sensor substrate 4. The sensor units 5 constitute, together with the pressure-sensitive adhesive layer 11, the lower surface of the sensor substrate 4. The sensor units 5 are of the same shapes as the shapes of the through holes 14, and have substantially cylindrical shapes.

Examples of materials of the sensor units 5 include metal conductors, conductive resins (including conductive polymers), and the like.

### (Battery-attached control unit)

The battery-attached control unit 6 includes a control unit 21 and a battery 22. The control unit 21 and the battery 22 are electrically connected to each other.

The control unit 21 is an integrated circuit that calculates and processes signals from the sensor units 5 and stores the signals. The control unit 21 includes an analog digital converter (ADC) 23, a microcomputer 24, memory 25, multiple terminals 26, and a wiring circuit board 27, which are electrically connected to each other.

The ADC 23 is a device for converting signals (analog signals) from the sensor units 5 into digital signals. For example, in a case where the adhering-type biosensor 2 is an electrocardiograph, changes of an electric potential (an electric signal) of the heart obtained by the sensor units 5 are converted into a digital signal.

The microcomputer 24 calculates the digital signal, and converts the digital signal into predetermined data. For example, in a case where the adhering-type biosensor 2 is an electrocardiograph, the digital signals are converted into electrocardiogram data of 16 bits and 1 kHz.

The memory 25 is a device that stores data calculated by the microcomputer 24, the digital signals from the ADC 23, and the like. For example, in a case where the adhering-type biosensor 2 is an electrocardiograph, the electrocardiogram data is stored to the memory 25.

Multiple (two) terminals 26 are elements that come into contact with multiple probes 9 of the external reception apparatus 3, explained later, to output internal data in the memory 25 to the external reception apparatus 3. Each of the terminals 26 is in a substantially rectangular shape in the plan view.

The multiple terminals 26 are arranged to be exposed from the control unit 21 toward the upper side. Specifically, the terminals 26 are arranged on the upper surface of the wiring circuit board 27, and the upper surface of each of the terminals 26 comes into contact with the coating layer 8.

The wiring circuit board 27 is a device that supports and fixes the ADC 23, the microcomputer 24, the memory 25, the terminal 26, and the battery 22 to electrically connect them with each other. The wiring circuit board 27 supports the ADC 23, the microcomputer 24, the memory 25, and the terminal 26 from the lower side, and supports the battery 22 from the upper side and the lower side. Specifically, the wiring circuit board 27 includes: a control unit support unit 27a of a substantially rectangular shape in the plan view that supports the ADC 23, the microcomputer 24, the memory 25, and the terminal 26; and a battery support unit 27b in an approximately circular shape in the plan view that is connected to the control unit support unit 27a and that supports the battery 22.

In the wiring circuit board 27, multiple conductive traces (not illustrated) electrically connecting the ADC 23, the microcomputer 24, the memory 25, the terminals 26, the battery 22, and the sensor wiring unit 7 (explained later) are formed, by way of vias, on the upper surface and the lower surface of the wiring circuit board 27.

The battery 22 has a disk shape or a button shape. The battery 22 includes terminals (a positive electrode terminal or a negative electrode terminal, not illustrated) on the upper surface and the lower surface, and each of the terminals is electrically connected by way of a lead wire (not illustrated) and the like to the wiring circuit board 27.

### (Sensor wiring unit)

The sensor wiring unit 7 is arranged on the upper surface of the sensor substrate 4. Specifically, the sensor wiring unit 7 is embedded in the substrate grooves 13 of the substrate layer 12 so that the upper surface of the sensor wiring unit 7 is exposed from the substrate layer 12.

The sensor wiring unit 7 includes multiple (two) conductive traces (sensor conductive traces) to electrically connect the sensor units 5 and the battery-attached control unit 6 with each other. Specifically, the sensor wiring unit 7 includes: a first conductive trace electrically connecting one of the sensor units 5 on one side in the second direction and the battery-attached control unit 6; and a second conductive trace electrically connecting the other of the sensor units 5 on the other side in the second direction and the battery-attached control unit 6.

Examples of materials of the sensor wiring unit 7 include conductors such as, for example, copper, nickel, gold, and alloys thereof, and the sensor wiring unit 7 is preferably made of copper.

The thickness of the sensor wiring unit 7 is, for example, thinner than the thickness of the substrate layer 12. Specifically, the thickness of the conductive trace is, for example, 0.1 µm or more, preferably 1 µm or more, and is, for example, 90 µm or less, preferably 45 µm or less.

### (Coating layer)

The coating layer 8 is a flexible member that protects and fixes the sensor unit 5, the battery-attached control unit 6, and the sensor wiring unit 7.

The coating layer 8 is arranged on the upper surface of the sensor substrate 4. Specifically, the coating layer 8 is arranged on the upper surface of the sensor substrate 4 so as to cover the upper surface and the side surfaces of the battery-attached control unit 6 and the upper surfaces of the sensor units 5 and the sensor wiring unit 7. In other words, the coating layer 8 completely seals, together with the sensor substrate 4, the battery-attached control unit 6 (the ADC 23, the microcomputer 24, the memory 25, the terminals 26, and the battery 22) and the sensor wiring unit 7, and further covers the upper surfaces of the sensor units 5. The coating layer 8 is bonded and fixed to the sensor substrate 4. The shape of the coating layer 8 in the plan view is substantially the same as the shape of the sensor substrate 4 in the plan view.

Examples of materials of the coating layer 8 include an insulator having elasticity. Examples of such materials include the same insulators as the materials of the sensor substrate 4, and the coating layer 8 is preferably made of a polyurethane-based resin.

The coating layer 8 has transparency. Therefore, the terminals 26 can be seen when the adhering-type biosensor 2 is seen from above.

The elastic modulus of the coating layer 8 is, for example, 1 GPa or less, preferably 150 MPa or less, more preferably 10 MPa or less, and is, for example, 1 MPa or more. When the elastic modulus is equal to or less than the above-described upper limit, the probes 9 can readily penetrate through the coating layer 8 to reach the terminals 26.

The thickness of the coating layer 8 is, for example, 1 µm or more, preferably 5 µm or more, and is, for example, 95 µm or less, preferably 50 µm or less.

On the upper surface of the terminal 26, a thickness T of the coating layer 8 is, for example, 0.1 mm or more, preferably 1 mm or more, and is, for example, 20 mm or less, preferably 5 mm or less. When the thickness T is equal to or more than the above-described lower limit, the terminals 26 can be reliably protected, and the terminals 26 can be prevented or inhibited from being damaged or exposed during measurement. When the thickness T is equal to or less than the above-described upper limit, the probes 9 can readily penetrate through the coating layer 8 to reach the terminals 26.

### 3. External reception apparatus

The external reception apparatus 3 is an apparatus configured to receive data from the adhering-type biosensor 2, and includes internal memory and multiple (two) probes 9 as illustrated in FIG. 2A.

The multiple probes 9 are terminals for retrieving data stored in the memory 25. Each of the probes 9 has an elongated conical shape (a needle shape), and the tips of the probes 9 are sharply pointed. The angles of the tips of the probes 9 are, for example, 120 degrees or less, preferably 90 degrees or less, and are, for example, 1 degree or more, preferably 5 degrees or more. When the angles of the tips are equal to or less than the above-described upper limit, the probes 9 can readily penetrate through the coating layer 8. When the angles of the tips are equal to or more than the above-described lower limit, the lengths of the probes 9 can be reduced, and the size of the external reception apparatus 3 can be reduced.

The multiple probes 9 are arranged to correspond to the multiple respective terminals 26. In other words, the interval between multiple probes 9 is substantially the same as the interval between the multiple terminals 26. Specifically, the multiple probes 9 are arranged so that, when one of the probes 9 comes into contact with one of the terminals 26, the other of the probes 9 can come into contact with the other of the terminals 26.

The other ends of the probes 9 are electrically connected to an external control unit (not illustrated), and the external control unit includes external memory (not illustrated) for storing data stored in the memory 25.

A length L of the probe 9 is longer than the thickness T. Specifically, for example, the length L is 0.1 mm or more, preferably 1 mm or more, and is, for example, 50 mm or less, preferably 10 mm or less.

The maximum diameter of the probe 9 is, for example, 0.05 mm or more, preferably 0.1 mm or more, and is, for example, 5 mm or less, preferably 1 mm or less.

### 4. Method for using bio-signal measurement system

The method for using the bio-signal measurement system 1, which is an example of a signal measurement system, is explained with reference to FIG. 2A and FIG. 2B. The method for using the bio-signal measurement system 1 is a method for obtaining data derived from a signal of a living body by using the bio-signal measurement system 1, and specifically, the method includes, in order, a preparation step, a measurement step, and a retrieval step.

### (Preparation step)

As illustrated in FIG. 2A, the preparation step includes preparing the bio-signal measurement system 1 by arranging the adhering-type biosensor 2 and the external reception apparatus 3.

### (Measurement step)

The measurement step includes pasting the adhering-type biosensor 2 to the living body, measuring the living body, and storing the data derived from the signal of the living body into the memory 25.

Specifically, first, the adhering-type biosensor 2 is pasted to the skin of the living body. In other words, the lower surface of the adhering-type biosensor 2 (the pressure-sensitive adhesive layer 11) is brought into contact with the skin of the living body. Accordingly, the adhering-type biosensor 2 is adhered (pasted) to the skin of the living body in a pressure-sensitive manner, and the sensor unit 5 comes into contact with the skin of the living body.

Subsequently, the living body is measured. In other words, the battery 22 is activated to give power to the control unit 21. Accordingly, in the adhering-type biosensor 2, the signal of the living body is detected by the sensor unit 5, the analog signal derived from the living body is transmitted to the ADC 23, and the ADC 23 converts the analog signal into a digital signal. The digital signal is calculated by the microcomputer 24 into desired data. Thereafter, the data is sequentially stored in the memory 25.

After the measurement is completed, the adhering-type biosensor 2 is detached from the living body.

In a case where the adhering-type biosensor 2 is an electrocardiograph, first, the adhering-type biosensor 2 is pasted to the chest of the living body and the battery 22 is activated. Accordingly, the electric signal of the heart (changes in the potential) is detected by the sensor unit 5, the electric signal (analog signal) thereof is transmitted to the ADC 23, and the ADC 23 converts the electric signal into a digital signal. The electric signal (the digital signal) of the heart is processed in calculation processing performed by the microcomputer 24 with a data rate of, for example, 16 bits and 1 kHz. Thereafter, the data of the electric signal is sequentially stored in the memory 25. After the measurement is completed, the adhering-type biosensor 2 is detached from the chest.

### (Retrieval step)

As illustrated in FIG. 2B, the retrieval step includes bringing the multiple probes 9 into contact with the multiple terminals 26.

Specifically, the multiple probes 9 are inserted into the coating layer 8 to penetrate through the coating layer 8. Then, the multiple probes 9 are brought into contact with the upper surface of the multiple terminals 26. As a result, the external memory and the memory 25 are electrically connected via the probes 9 and the terminals 26, and the data stored in the memory 25 is transmitted to the external memory.

Subsequently, as necessary, the data received in the external memory is retrieved into another computer by a known method, the data is displayed on the computer screen, and the data is seen.

In a case where the adhering-type biosensor 2 is an electrocardiograph, the data stored in the memory 25 is transmitted to the external memory by bringing the multiple probes 9 into contact with the multiple terminals 26. Thereafter, as necessary, the data received in the external memory is displayed as an electrocardiogram waveform on another computer screen, and the electrocardiogram waveform is seen.

### 5. Application of bio-signal measurement system

The biosensor 2 is not particularly limited so long as the biosensor 2 is a device that can measure the state of, for example, the living body by detecting an electric signal from the living body. Specifically, examples of applications of the biosensor 2 include an adhering-type electrocardiograph, an adhering-type electroencephalograph, an adhering-type sphygmomanometer, an adhering-type pulse rate monitor, an adhering-type electromyogram, an adhering-type thermometer, an adhering-type accelerometer, and the like. Further, each of these devices may be an individual device, or a plurality thereof may be incorporated in a single device.

The adhering-type biosensor 2 is preferably used as an adhering-type electrocardiograph. In the adhering-type electrocardiograph, the sensor unit 5 senses the action potential of the heart as an electric signal.

The living body may be a human body or organisms other than humans (animals, plants). However, the living body is preferably a human body.

According to the bio-signal measurement system 1 and the data acquisition method using the same, the probes 9 of the external reception apparatus 3 are inserted through the coating layer 8 to be in contact with the terminals 26, so that the data in the memory 25 is retrieved. Therefore, the data derived from the living body can be obtained easily.

In addition, the wired method in which the probes 9 are in contact with the terminals 26 is adopted, so that the communication speed and communication accuracy are better than those of the wireless method. Therefore, data can be obtained reliably.

### 6. Modified embodiment

In the following modified embodiments, members and steps similar to those of the above embodiment are denoted with the same reference numerals, and detailed description thereabout is omitted. Also, the modified embodiments may be combined as appropriate. Further, the modified embodiment can achieve the same actions and effects as those of the above embodiment, unless otherwise specified.

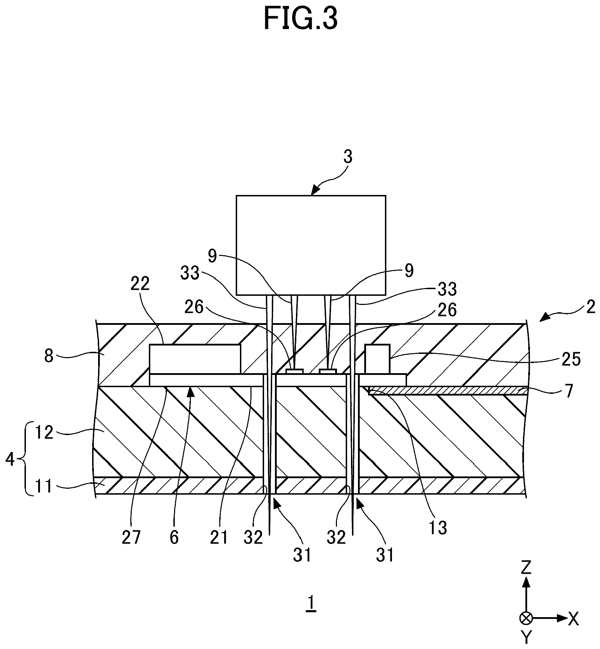

(1) In the bio-signal measurement system 1 as illustrated in FIG. 1A, the adhering-type biosensor 2 does not have any alignment unit. However, for example, as illustrated in FIG. 3, the adhering-type biosensor 2 may include multiple (two) alignment units 31.

The multiple alignment units 31 include through holes 32 that penetrate through the control unit 21 and the sensor substrate 4 in the thickness direction. The through holes 32 have approximately circular shapes in the plan view. The diameters of the through holes 32 are formed so as to be equal to or more than the diameters of the tips of the probes 9.

In this embodiment, multiple guide pins 33 corresponding to multiple alignment units 31 are provided.

The length of the guide pins 33 are longer than the lengths of the probes 9 and are larger than the thickness of the adhering-type biosensor 2.

The multiple guide pins 33 are arranged so as to correspond to the multiple alignment units 31. In other words, the interval between the multiple guide pins 33 is substantially the same as the interval between the multiple alignment units 31. Specifically, the multiple guide pins 33 are arranged so that, when one of the guide pins 33 is inserted into one of the alignment units 31, the other of the guide pins 33 can be inserted into the other of the alignment units 31.

Also, the multiple guide pins 33 and the multiple probes 9 are arranged to correspond to the multiple alignment units 31 and the multiple terminals 26, respectively. Specifically, the multiple guide pins 33 and the multiple probes 9 are arranged so that, after the multiple guide pins 33 are inserted into the multiple alignment units 31, the multiple probes 9 can come into contact with the multiple terminals 26.

In the configuration example as illustrated in FIG. 3, the alignment units 31 covered with the coating layer 8 can be easily seen. With the alignment units 31 serving as a reference, the probes 9 can be easily brought into contact with the terminals 26.

The number of alignment units 31 is not limited. There may be one alignment unit 31, or there may be three or more alignment units 31.

(2) In the bio-signal measurement system 1 as illustrated in FIG. 2A and FIG. 2B, the external reception apparatus 3 includes two probes 9, with one of the probes 9 being brought into contact with a corresponding one of the terminals 26. However, the number of probes 9 brought into contact with a single terminal 26 is not limited.

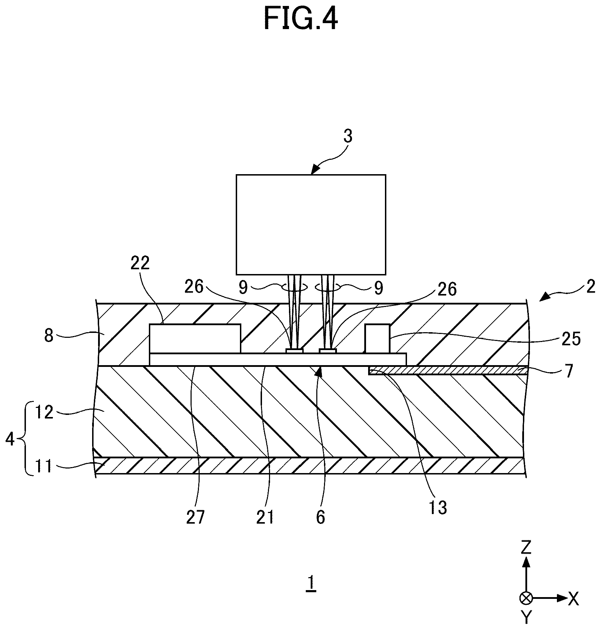

For example, as illustrated in FIG. 4, four probes 9 may be provided, and multiple (two) probes 9 of the four probes 9 can also be brought into contact with a single terminal 26.

Also, the number of terminals 26 and probes 9 is not limited, and each of them may be one or may be three or more.

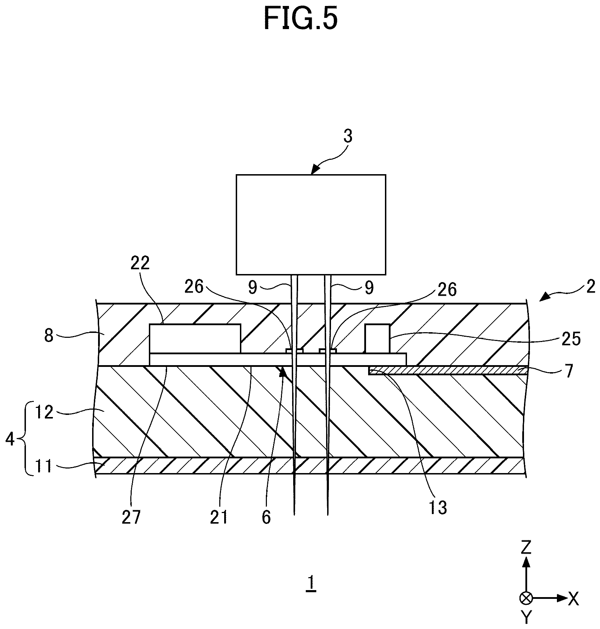

(3) In the data acquisition method as illustrated in FIG. 2A and FIG. 2B, the probes 9 are brought into contact with the terminals 26 so that the probes 9 are arranged on the upper surfaces of the terminals 26. However, for example, as illustrated in FIG. 5, the probes 9 can also be brought into contact with the terminals 26 in such a manner that the probes 9 penetrate through the terminals 26 and the sensor substrate 4.

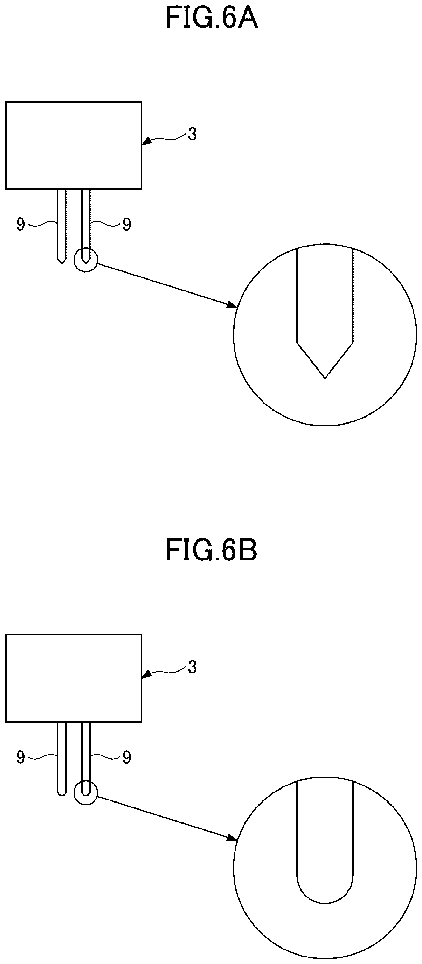

(4) In the external reception apparatus 3 as illustrated in FIG. 2A and FIG. 2B, the probes 9 may have needle shapes (conical shapes). However, the probes 9 may have polygonal pyramid shapes such as triangular pyramid shapes. Alternatively, as illustrated in FIG. 6A and FIG. 6B, the probes 9 may have long plate-like shapes with sharp tips. In this embodiment, the tips of the probes 9 may be, for example, in a triangular shape as illustrated in FIG. 6A, an arc shape as illustrated in FIG. 6B, and the like.

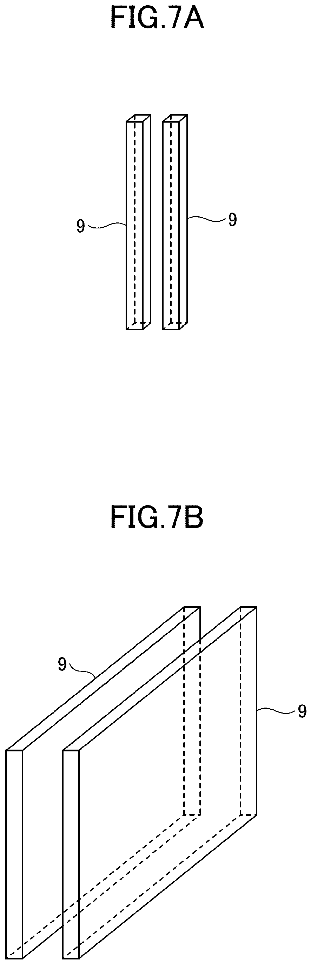

(5) In the external reception apparatus 3 as illustrated in FIG. 2A and FIG. 2B, the probes 9 have sharp tips. However, for example, as illustrated in FIG. 7A and FIG. 7B, the probes 9 may have shapes in which the tips are not sharp (a flat shape). For example, in this embodiment, the probes 9 may have rectangular shapes (square rod shapes) extending in the vertical direction as illustrated in FIG. 7A, or may have rectangular shapes (strip-like shapes) extending in a planar direction including the vertical direction as illustrated in FIG. 7B. From the perspective of the ease of penetration, the aspects as illustrated in FIG. 2A, FIG. 2B, FIG. 6A, and FIG. 6B may be adopted. From the perspective of the ease of contact with the terminals 26, the aspects as illustrated in FIG. 7A and FIG. 7B can be adopted.

(6) In the adhering-type biosensor 2 as illustrated in FIG. 1A, the control unit 21 and the battery 22 are provided as a single component (the battery-attached control unit 6). However, for example, these may be provided as separate members, although not illustrated. In this embodiment, the control unit 21 and the battery 22 are electrically connected via the sensor wiring unit 7.

### <Second embodiment>

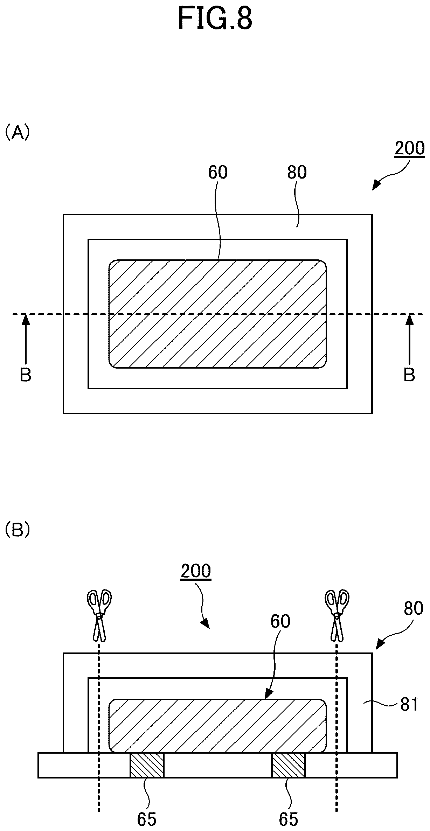

FIG. 8 is a schematic diagram of a biosensor 200 according to the second embodiment. In the first embodiment, the wiring circuit board 27 on which the components are mounted is covered with the coating layer 8. In the second embodiment, the biosensor 200 is formed to be openable, and a sensor chip 60 is arranged in a casing 80 in a removable manner. The sensor chip 60 is an example of a device for measuring various data and storing the data in the memory, and is a target of data-reading.

(A) of FIG. 8 is a schematic plan view of the biosensor 200. (B) of FIG. 8 is a cross-sectional view taken along line B-B of (A) of FIG. 8.

The sensor chip of the biosensor 200 is placed in the internal space of the casing 80, and a cover 81 can be easily removed by cutting it with scissors, for example. Similarly with the first embodiment, electrodes functioning as sensor units 65 are arranged so as to be exposed on the back surface of the casing 80. The sensor chip 60 is taken out from the casing 80 after the data measurement, and the measurement data is read out.

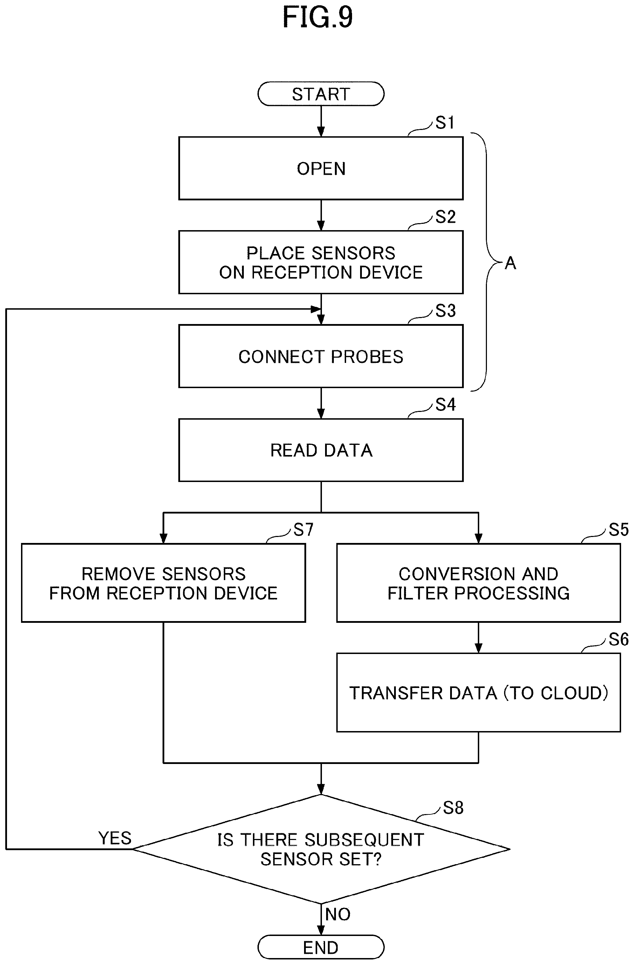

FIG. 9 is a flowchart of a data acquisition method according to the second embodiment. First, the casing 80 of the biosensor 200 is opened to take out the sensor chip 60 (S1). The opening of the casing 80 and the removal of the sensor chip 60 may be performed manually by an operator or automatically by a robot arm or the like. It takes approximately one minute or less than one minute to complete the removal work of the sensor chip 60.

The removed sensor chip 60 is placed on a reception device (S2). The sensor chip 60 may be placed on the reception device manually by an operator or automatically by an automatic conveyance device such as a robot arm.

The reception device may be configured to accommodate a single sensor chip 60. However, in a preferred configuration example, multiple sensor chips are accommodated, and data is read out in parallel. It takes approximately one minute or less than one minute to complete the placement work of the sensor chip 60 in the reception device.

The probes are connected to the reading terminals of the sensor chip 60 (S3). The terminals are, for example, conductive pads arranged on the surface of the sensor chip 60. It takes approximately one minute or less than one minute to complete the connection work to connect the probes to the terminals of the sensor chip 60.

In a case where multiple sensor chips 60 are placed on the reception device, multiple probes are connected to each of the sensor chips 60. With the use of a probe mechanism explained later, multiple probes can be connected to the conductive pads of the corresponding sensor chips 60 substantially at the same time.

Steps S1 to S3 are a preparation step A for reading data, and it takes totally about three minutes to perform steps S1 to S3.

Subsequently, with the probes, the measurement data is read out from the sensor chip 60 (S4). It takes approximately one minute to read the measurement data. The data read out from each of the sensor chips 60 is processed in conversion processing, filter processing, and the like (S5) in parallel by an information processing apparatus such as a personal computer (PC). It takes about three minutes to perform the data processing.

The data processed by the information processing apparatus may be transferred to the outside of the system, for example, transferred to a server, a data center, a cloud, and the like (S6). For example, it takes about five minutes to perform this transfer step S6. The step of data processing (S5) and the step of transfer of the processed data (S6) may be performed in parallel at the same time. The transfer of the data to an external apparatus is not mandatory, and the converted and processed data may be temporarily stored in internal memory of a PC and the like.

When the reading of the data (S4) is completed, the sensor chip 60 is ready to be removed from the reception device (S7). It takes approximately one minute or less than one minute to perform the removal work of the sensor chip 60. During data processing such as conversion and filtering (S5), sensor chips 60 of which the reading has been completed may be removed and a subsequent set of sensor chips 60 may be set.

After the completion of reading of the data (S4), a determination is made as to whether a subsequent set of sensor chips has been placed on the reception device (S8). Whether there is a subsequent set of sensor chips may be determined by, for example, information about the placement state of each of the sensor chips 60 from the reception device. Alternatively, a set position of each of the sensor chips 60, whether there is a sensor chip 60 at the set position, a setting error, and the like may be determined on the basis of changes in the pressure, mass, electric capacitance, and the like in each placement state on the reception device.

In a case where a subsequent set of sensor chips is placed (YES in S8), the flow returns back to step S3 to repeat S3 to S7 on subsequent sensor chips. The reading of data from the subsequent sensor chips (S4) is desired to be started at a point in time when the conversion and filter processing of the previous sensor chips has been completed. In a case where a subsequent sensor set is not placed for a predetermined period of time or more (NO in S8), the processing is terminated.

In the data acquisition method of FIG. 9, reading of data from multiple sensor chips is performed in parallel, and the processing performed on the read data is performed in parallel, so that the number of times the data acquisition work is performed is reduced. In addition, at a point in time when the data reading (S4) is completed, the sensor chips 60 are removed from the reception device and subsequent sensor chips 60 are set, so that reading of data can be started immediately after the conversion and filter processing (S5), and therefore, the processing time is further reduced.

The time required for each step described above is an example, and varies slightly depending on the memory capacity of the sensor chip 60 and the like. Even in such a case, at a point in time when the data processing of the obtained data is completed, reading of data from a subsequent set of sensor chips can be started in parallel.

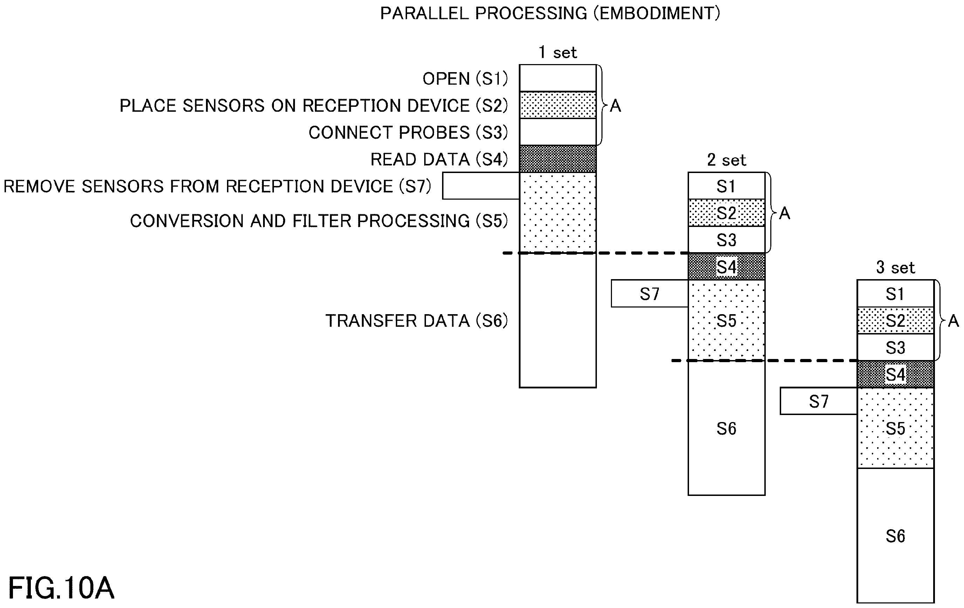

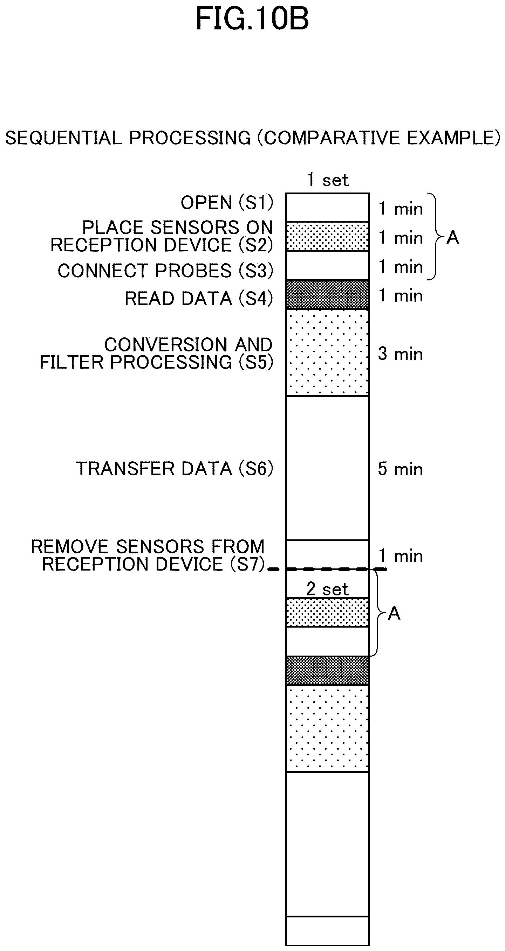

FIG. 10A illustrates parallel processing during data acquisition according to the second embodiment. FIG. 10B illustrates generally-available sequential processing as a comparative example. In FIG. 10A, at a point in time when reading of data of the first set of sensor chips (S4) is completed, the first set of sensor chips is removed from the reception device. At this point in time, the second set of a biosensor 200 has been opened, and the second set of sensor chips 60 has been taken out from the package. The opening of the second set of the biosensor 200 and the removal of the sensor chips 60 (S1) may be performed during or before the conversion and filter processing (S5) for the first set.

When the first set of sensor chips 60 is removed from the reception device, the second set of sensor chips 60 is set on the reception device (S2), and the probes are connected (S3). Immediately after the completion of the conversion and filter processing (S5) of the data read out from the first set of sensor chips 60, reading of data from the second set of sensor chips 60 (S4) is started. In a case where the second set includes multiple sensor chips 60, reading of the data is performed in parallel.

During transfer of data that has been read out from the first set (S6), the conversion and filter processing (S5) of the data that has been read out from the second set of sensor chips 60 is performed.

Likewise, when reading of the data from the second set of sensor chips 60 (S4) has been completed and the second set of sensor chips 60 is removed from the reception device, the third set of sensor chips 60 is placed on the reception device and the probes are connected. Immediately after the conversion and filter processing (S5) for the second set is completed, parallel reading processing for reading data from the third set of sensor chips 60 is started.

According to this configuration and method, the waiting time is minimized and efficient data reading is achieved.

In contrast, in the sequential processing of FIG. 10B, steps S1 to S7 are performed in order. When reading of data (S1) from the first set is completed, it is possible to remove the first set of sensor chips from the reception device. However, reading of data from the sensor chips for the second set of sensor chips starts after the procedure for the first set has been completed, and as a result, a waiting time occurs. Even if data is read out from multiple sensor chips in parallel, the point in time when data is read from the subsequent set of sensor chips is delayed. The reading of data according to the second embodiment solves inefficient data reading of FIG. 10B.

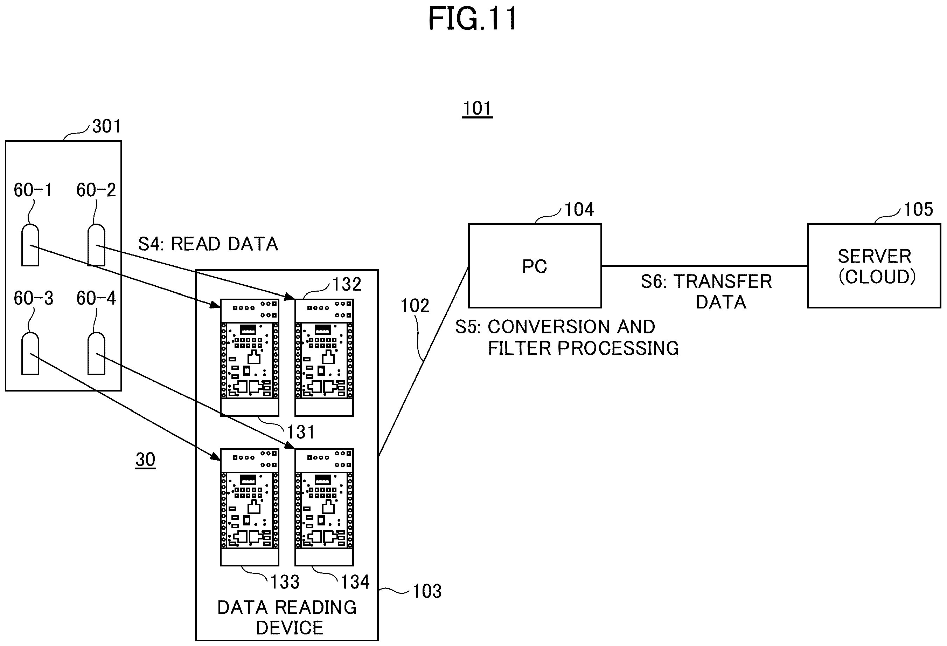

FIG. 11 is a schematic diagram of a bio-signal measurement system 101 according to the second embodiment. The bio-signal measurement system 101 includes a data reading apparatus 30 and an information processing apparatus 104. The data reading apparatus 30 corresponds to the external reception apparatus 3 according to the first embodiment, and reads data from the biosensor 200. A server 105 and the like for receiving transfer of data from the information processing apparatus 104 may be included in the bio-signal measurement system 101.

The data reading apparatus 30 includes a reception device 301 and a data reading device 103. The data reading device 103 is connected to a probe mechanism for data reading as explained later.

The reception device 301 is configured so as to allow multiple sensor chips 60 to be placed. In this example, four sensor chips, i.e., sensor chips 60-1 to 60-4, are placed, but the number of sensor chips that can be placed is not limited to four.

As described above, the reception device 301 may generate information indicating whether there is a sensor chip 60 at each sensor chip placement position and transmit the information to the data reading device 103. The reception device 301 may have means for displaying, if an error occurs when sensor chips 60 are set, information about a sensor chip 60 with which the error has occurred or information about the position where that sensor chip 60 is placed. By displaying the information about the placement error, it is possible to prompt an operator to place the sensor chips 60 again. Together with the display of he information about the placement error, the error information may be output to the data reading device 103, the information processing apparatus 104, and the like.

The data reading device 103 includes control circuits 131 to 134 according to the number of sensor chips 60 placed on the reception device 301. The control circuits 131 to 134 read, in parallel, data from the sensor chips 60-1 to 60-4 via the probe mechanism explained later, and transmit the read data into the information processing apparatus 104 via a bus 102 such as a USB. The data reading device 103 may be provided with a host control circuit for controlling the entire operation of the control circuits 131 to 134.

When supplying of the data to the information processing apparatus 104 has been completed, the control circuits 131 to 134 may output, to the reception device 301, a signal indicating that the sensor chips 60-1 to 60-4 are in a state of being ready to be removed. The completion of the data processing may be notified to the reception device 301 by causing the probe mechanism, which has been in contact with the sensor chips 60-1 to 60-4, to be made into non-contact therewith. Thereafter, information indicating whether a subsequent set of sensor chips 60 has been set may be obtained from the reception device 301.

When the subsequent set of sensor chips 60 is set on the reception device 301, the probes are connected to the sensor chips 60, and reading of subsequent data is automatically started. During this period, the information processing apparatus 104 may transfer the processed data to, for example, the external server 105, a cloud, a data center, and the like. In other words, during the data transfer by the information processing apparatus 104, reading of the data and the data processing for the subsequent set can be performed, so that the waiting time can be minimized.

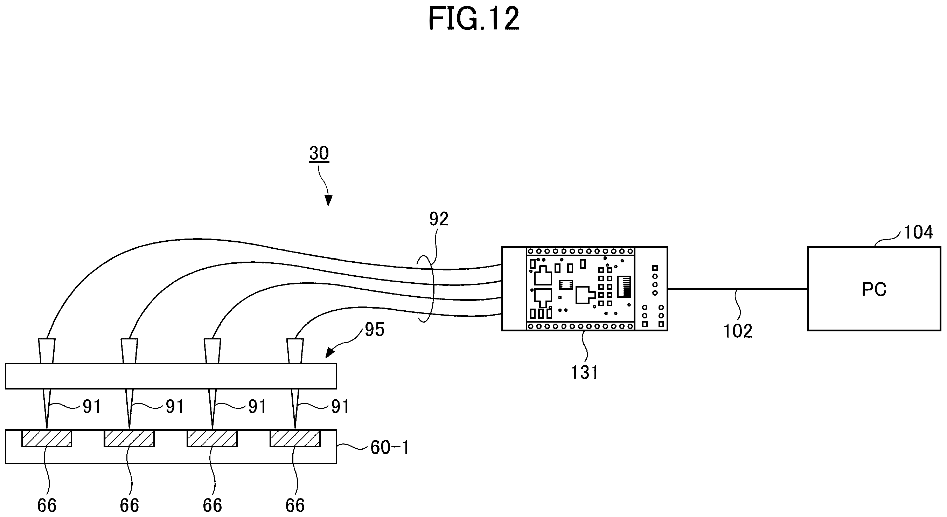

FIG. 12 is a schematic diagram of a probe unit 95 used for the second embodiment. For example, the probe unit 95 is formed as a pin board, and is connected by wires 92 to a control circuit 131. The probe unit 95 may have pins 91 according to the number of conductive pads 66 of the sensor chip 60-1.

The data read with the probe unit 95 is transmitted by the control circuit 131 to the information processing apparatus 104 via the bus 102.

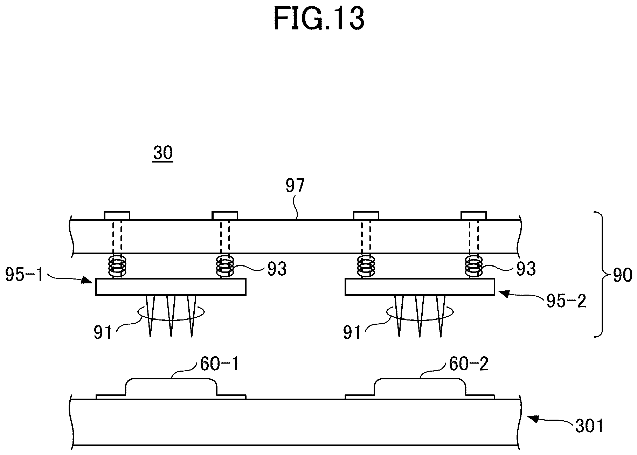

FIG. 13 is a schematic diagram of a probe mechanism 90 for performing parallel reading. The probe mechanism 90 includes multiple probe units 95-1 and 95-2 held on a circuit board 97. The probe units 95-1 and 95-2 read data from the sensor chips 60-1 and 60-2 placed on the reception device 301. Each of the probe units 95 is connected to the circuit board 97 by a wire, not illustrated, and the like, and the circuit board 97 is connected to the data reading device 103 (see FIG. 11).

Each of the probe units 95 with the pins 91 may be attached to the circuit board 97 by elastic components 93 such as springs. The springs allow the probe units 95 to be movable, so that the tips of the pins 91 can be pressed against the conductive pads 66 on the corresponding sensor chips 60. By ensuring the contact between the pins 91 and the sensor chips 60, the reliability of parallel reading of data can be improved.

### <Other configuration examples>

In the first embodiment and the second embodiment, the bio-signal measurement system and the method for using the same (the data acquisition method) for measuring the living body has been explained as an example of a signal measurement system and a method for using the same according to the present invention. However, the signal measurement system and the method for using the same according to the present invention are not limited thereto. For example, it can also be used for a signal measurement system and a method for using the same for measuring home appliances and electronic devices, building components, transportation devices, films (optical films, packaging films, and the like), and the like. The biosensor according to the first embodiment can also be processed in parallel similarly with the second embodiment.

This application is based on and claims priority to Japanese Patent Application No. 2018-196787 filed on October 18, 2018 and Japanese Patent Application No. 2019-177231 filed on September 27, 2019, the entire contents of which are incorporated herein by reference.

### REFERENCE SIGNS LIST

- 1, 101: bio-signal measurement system
- 2, 200: biosensor
- 3: external reception apparatus
- 5: sensor unit
- 8: coating layer
- 9: probe
- 25: memory
- 26: terminal
- 30: data reading apparatus
- 31: alignment unit
- 50: sensor electrode
- 60, 60-1 to 60-4: sensor chip (device)
- 66: conductive pad
- 80: casing
- 81: cover
- 82: bottom plate
- 90: probe mechanism
- 91: pin
- 95: probe unit
- 102: bus
- 103: data reading device
- 104: information processing apparatus
- 105: server
- 131, 132, 133, 134: reading control circuit
- 301: reception device

### Citation List

### Patent Literature

PTL 1: Japanese Laid-Open Patent Publication No. 2012-10978

## Claims

1. A data acquisition method for obtaining data from a sensor including a sensor unit configured to sense a physical or electric signal, memory configured to store, as the data, the physical or electric signal from the sensor unit, a terminal configured to output the data in the memory, and a cover covering the memory and the terminal,
the data acquisition method comprising:
preparing an external apparatus including a probe for retrieving the data from the memory; and
retrieving the data stored in the memory by bringing the probe of the external apparatus in contact with the terminal.

2. The data acquisition method according to claim 1, wherein the cover is a coating layer covering the memory and the terminal, and
the probe penetrates through the coating layer to be in contact with the terminal.

3. The data acquisition method according to claim 1, wherein the cover is a portion of a casing that is openable,
the memory and the terminal are provided in a device accommodated in the casing, and
the probe is brought into contact with the terminal of the device taken out from the casing.

4. The data acquisition method according to claim 1, wherein the external apparatus includes a probe mechanism configured to retrieve, in parallel, the data from a plurality of devices and a control circuit configured to control reading of the data from the plurality of devices with the probe mechanism, and
at a point in time when data processing on the data read in parallel with the probe mechanism is completed, the control circuit starts reading, in parallel, data from a subsequent device set.

5. The data acquisition method according to claim 4, wherein during transfer of the data on which the data processing has been performed, the control circuit performs data processing on the data read from the subsequent device set.

6. A signal measurement system comprising:
a reception device on which a plurality of devices storing measurement data are placed;
a probe mechanism configured to be able to come into contact with the plurality of devices placed on the reception device;
a control circuit configured to read, in parallel, the measurement data from the plurality of devices with the probe mechanism; and
an information processing apparatus configured to process, in parallel, the measurement data having been read,
wherein at a point in time when a subsequent device set is placed on the reception device, the control circuit drives the probe mechanism, and
at a point in time when data processing with the information processing apparatus is completed, the control circuit starts reading, in parallel, data from the subsequent device set.

7. The signal measurement system according to claim 6, wherein the reception device generates and outputs information about a placement state of the plurality of devices, and
the control circuit drives the probe mechanism based on the information.

8. The signal measurement system according to claim 7, wherein the information includes at least one of a placement position of each of the plurality of devices, whether one of the plurality of devices is present at the placement position, and a placement error.

9. The signal measurement system according to any one of claims 6 to 8, wherein one of the plurality of devices is a sensor chip configured to obtain data derived from a signal of a living body in such a manner that the sensor chip is accommodated in a casing and that the casing is brought into contact with the living body, and is taken out from the casing and placed on the reception device.

10. The signal measurement system according to any one of claims 6 to 8, wherein one of the plurality of devices is a biosensor configured to sense a signal from a living body by being brought into contact with the living body, and
the biosensor further includes an alignment unit serving as a reference of alignment of the probe mechanism with respect to the biosensor.
